# EUROPEAN PATENT APPLICATION

(11) **EP 0 742 008 A1**
(43) Date of publication of application: **13.11.1996**
(21) Application number: 95107121.6
(22) Date of filing: 11.05.1995
(51) Int. Cl.: A61K 31/135, A61K 9/16, A61K 9/20, A61K 9/28, A61K 9/50

(54) **Controlled release pharmaceutical formulations**

(71) Applicant: Euro-Celtique S.A., Luxemburg (LU)
(72) Inventor: Bräuninger, Renate, Dr., 65510 Idstein (DE); Heun, Gerhard, 65611 Niederbrechen (DE); Winkler, Horst, Dr., 65550 Limburg (DE)
(74) Representative: Meyers, Hans-Wilhelm, Dr.Dipl.-Chem.

(57) **Abstract**

A solid, oral controlled release dosage form containing as active ingredient a pharmaceutically active compound which contains a 2-amino-1-hydroxyethyl-substituted benzene structure, wherein the amount of the initial pharmaceutically active compound which degrades after storage of the dosage form for three months at 40°C and 75% relative humidity is less than 1.5%.

## Description

This invention relates to solid, oral, controlled release dosage forms which contain a pharmaceutically active ingredient having a 2-amino-1-hydroxyethyl-substituted benzene structure.

Oral formulations containing pharmaceutically active ingredients having a 2-amino-1-hydroxyethyl-substituted benzene structure have been known for many years as most of the commercial β₂-sympathomimetics fall into this category. Examples of such compounds are Salbutamol, Terbutalin, Carbuterol, Clenbuterol, Fenoterol and Reproterol. These compounds are, however, sensitive to oxidation and as a result, most solid preparations of 2-amino-1-hydroxy-substituted benzenes contain degradation products which are mainly the ketone derivatives formed by oxidation of the hydroxy group of the 1-hydroxyethyl moiety.

It was unexpectedly found that by appropriate selection of the controlled release material used in conjunction with 2-amino-1-hydroxyethyl benzene compounds it is possible to obtain preparations in which the active ingredient shows a surprising degree of chemical stability.

In accordance with one aspect of the invention, therefore, a solid, oral, controlled release dosage form is provided containing as active ingredient a pharmaceutically active compound which contains a 2-amino-1-hydroxyethyl-substituted benzene structure in association with a controlled release material, wherein the amount of the initial pharmaceutically active compound which degrades after storage of the dosage form for three months at 40°C and 75% relative humidity (rh) is less than 1.5%, preferably less than 1% and more preferably less than 0.6% or 0.5%.

A preferred formulation in accordance with the invention shows a degradation of the aforesaid active ingredient of approximately 0.25% or less under the aforesaid storage conditions after one month's storage. Another preferred formulation shows a degradation of the active ingredient of no more than 1% after storage at 25°C and 60% rh.

The dosage form in accordance with the invention may be presented as, for example, granules, spheroids or pellets in a capsule or in any other suitable dosage form such as a tablet.

A preferred dosage form in accordance with the invention is in the form of a controlled release matrix with the pharmaceutically active compound dispersed therein.

The present invention is predicated on the finding that by appropriate selection of the controlled release material with which the active ingredient is to be associated, it is possible to incorporate the active ingredient in an environment in which it is significantly less susceptible to degradation than with other, conventionally used controlled release material, whilst at the same time providing for satisfactory control of the release rate. In particular, it was unexpectedly found that widely used controlled release materials such as hydroxypropylmethylcellulose (HPMC), hydrogenated oils, e.g. hydrogenated castor oil, and microcrystalline cellulose, are implicated in the accelerated degradation of 2-amino-1-hydroxyethyl-substituted benzenes. A controlled release dosage form in accordance with the invention will, therefore, as a general rule, not contain any cellulose derivative or hydrogenated oil derivative such as hydrogenated castor oil or will be so constructed that the said active ingredient is not in contact with a cellulose derivative of hydrogenated oil derivative.

One of the most successful and widely used controlled release substrates comprises a combination of a C₈ - C₅₀ especially C₁₂ - C₄₀, and suitably C₈ -C₁₈ aliphatic alcohol optionally substituted with a C₈ - C₁₈ alkyl group and a cellulose derivative such as hydroxypropylmethylcellulose. A controlled release preparation sold under the Registered Trade Mark MST CONTINUS comprises a matrix of cetostearyl alcohol and hydroxypropylmethylcellulose in which the active ingredient is morphine sulphate.

It has been found, in accordance with a preferred aspect of the present invention, that it is particularly advantageous to use a controlled release matrix containing an C₈ - C₅₀, especially C₁₂ - C₄₀, suitably C₈-C₁₈, aliphatic alcohol optionally substituted by an aliphatic group containing 8 to 18 carbon atoms, preferably those having a melting point of 25°C and 90°C, as the sole release controlling excipient and it has been surprisingly found that such alcohols, in the absence of any cellulose derivative, e.g. hydrogxypropylmethylcellulose, with which they are conventionally used to provide controlled release matrices, nonetheless gives a controlled release of the 2-amino-1-hydroxyethyl benzene compound allowing e.g. for twice-a-day or once-a-day dosing. Suitable aliphatic alcohols are lauryl alcohol, myristyl alcohol, cetyl alcohol, stearyl alcohol and cetostearyl alcohol.

Even more surprisingly it has been found that the aliphatic alcohol may be combined with a water soluble diluent or excipient such as a saccharide e.g. a disaccharide such as lactose, or a water insoluble diluent or excipient such as dicalcium phosphate to provide a controlled release preparation in accordance with the invention. In a preferred embodiment a controlled release matrix consists essentially of an aliphatic alcohol (a.a) and saccharide (s) present in the relative proportions by weight (a.a:s) of 1:1.5 to 1:6 e.g. (1.75 to 1):5 and most preferably 37:63 to 16:84 or an aliphatic alcohol (a.a) and dicalcium phosphate (dcp) are present in the relative proportions by weight of a.a:dcp of 1:1.5 to 1:2.5 e.g. 1:1.7 to 1:2.1 and most preferably 37:63 to 32:68. Combinations of aliphatic alcohol, saccharide and dicalcium phosphate are also contemplated for matrices in accordance with the invention.

In addition to the ingredients mentioned above, a controlled release matrix in a dosage form in accordance with the invention may also contain suitable quantities of other materials e.g. diluents, lubricants, binders, granulating aids, colourants, flavourants and glidants that are conventional in the pharmaceutical art.

In order to facilitate the preparation of a solid, controlled release, oral dosage form according to this invention there is provided, in a further aspect of the present invention, a process for the preparation of such a solid, controlled release, oral dosage form, comprising bringing a pharmaceutically active compound having a 2-amino-1-hydroxyethyl-substituted benzene structure into association with a controlled release material to form solid, oral, controlled release preparation in accordance with the invention.

A preferred method in accordance with the invention comprises incorporating the pharmaceutically active compound in a suitable controlled release matrix.

Incorporation in the matrix may be effected, for example, by forming granules comprising at least one digestible C₈ -C₅₀ substituted or unsubstituted fatty alcohol and, as described above, a pharmaceutically active 2-amino-1-hydroxyethyl compound e.g. by mixing the ingredient with the fatty alcohol at a temperature and speed at which the fatty alcohol melts or softens and granules are formed.

The granules may also be formed by wet granulation of the pharmaceutically active material and a water soluble or water insoluble excipient e.g. a saccharide such as lactose or dicalcium phosphate and mixing the resulting granules with at least one digestible C₈ -C₅₀ substituted or unsubstituted fatty alcohol.

The granules may be in the form of spherical or spheroidal pellets e.g. 0.2 mm to 2.5 mm in diameter or small, irregularly shaped particles.

The granules so formed are optionally compressed and shaped e.g. to form tablets, or may be encapsulated.

Preferred formulations in accordance with the invention suitable for twice-a-day dosing contain 2 to 6% w/w e.g. 2.5 to 5.8% w/w of a pharmaceutically active compound having a 2-amino-1-hydroxyethyl-benzene structure, 10 to 40% w/w e.g. 14 to 32% w/w of a C8 - C50 substituted or unsubstituted fatty alcohol and 50 to 80% w/w e.g. 53 to 73% w/w water soluble or water insoluble diluent or carrier. More preferred is a matrix formulation containing 2.5 to 5.8% w/w salbutamol; 14 to 32% w/w of cetostearylalcohol and 53 to 72% w/w of lactose and/or dicalcium phosphate.

A preferred dosage form in accordance with the invention, having suitably a formulation as in the preceding paragraph has a dissolution rate of the active ingredient when measured according to EP Paddle Dissolution System using 450 ml water, a temperature of 37°C, a paddle speed of 100 rpm and detection with UV at 225 nm, of 20 - 40% at one hour, 30 - 60% at two hours, 50 - 80% at four hours, 75 - 95% at eight hours and more than 85% at ten hours.

The presence and amount of degradation products can be readily determined using conventional methods. Once such method utilizes HPLC using a stationary phase LiChrospher RP-8 (125 x 4 mm, 5 µm), as mobile phase water/acetonitrile - 0.1 M H₃PO₄, gradient, with detection at 210 nm and using a standard solutiong with a concentration of 100 mg/100 ml, a reference solution with a concentration of 0.5 mg/100 ml and a test solution with a concentration of 100 mg/100 ml.

### Examples

| **Formulation** | **mg/Tablet** |
|---|---|
| Salbutamol sulphate | 4.82 |
| Lactose monohydrate | 63.28 |
| Cetostearyl alcohol | 28.80 |
| Magnesium stearate | 1.20 |
| Purified talc | 1.90 |
| TOTAL | 100.00 |

The tablets were made according to the above formulation as follows:

A mixture of the salbutamol sulphate and the lactose monohydrate was granulated in a high shear mixer whilst adding the cetostearylalcohol in molten form and the whole was mixed thoroughly. The resulting granules were milled and the purified talc and magnesium stearate were added and blended with milled particles. The granules were then compressed into tablets.

### Comparative Example

| **Formulation** | **mg/Tablet** |
|---|---|
| (1) Matrix | |
| Salbutamol sulphate | 9.64 |
| Lactose monohydrate | 113.16 |
| Microcrystalline cellulose | 13.20 |
| Methylhydroxypropylcellulose (HPMC) | 66.00 |
| Macrogol 4000 | 5.50 |
| Magnesium stearate | 0.50 |
| TOTAL | 208.00 |

| (2) *Film Coating* | |
|---|---|
| Methylhydroypropylcellulose | 16.14 |
| Talc | 1.62 |
| Macrogel 6000 | 3.22 |
| Magnesium stearate | 3.22 |

The tablets were made according to the above formulations as follows:

A mixture of the salbutamol sulphate, the lactose, microcrystalline cellulose and about 90% of the HPMC was granulated in a high shear mixer with a solution of Macrogol 4000 in purified water and transferred to a fluid bed drier. After drying, the granules were milled by passage through a screen. The remaining quantity of HPMC and the magnesium stearate was added and blended with the granules. The blended mixture was then compressed into tablets.

The resulting tablets were coated with a suspension of the film coating formulation given above.

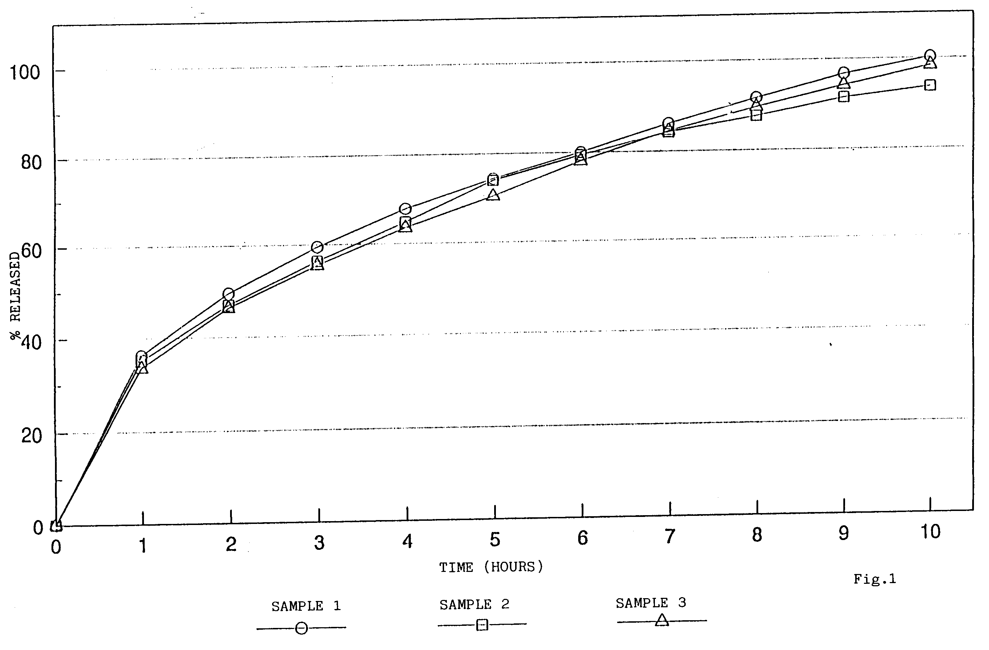

Dissolution tests according to the method described above were carried out on three samples of tablets made as described above and the results are shown in Figure 1. These results indicate that such tablets would be suitable for twice-a-day dosing.

Tablets made in accordance with the Example of Comparative Example were tested at twice monthly periods for degradation products after storage under the following conditions:
25°C/60% relative humidity (rh)
30°C/60% rh
40°C/70% rh
and for the Comparative Example the amount of degradation product after storage at 21°C for 11 and 14 months was tested.

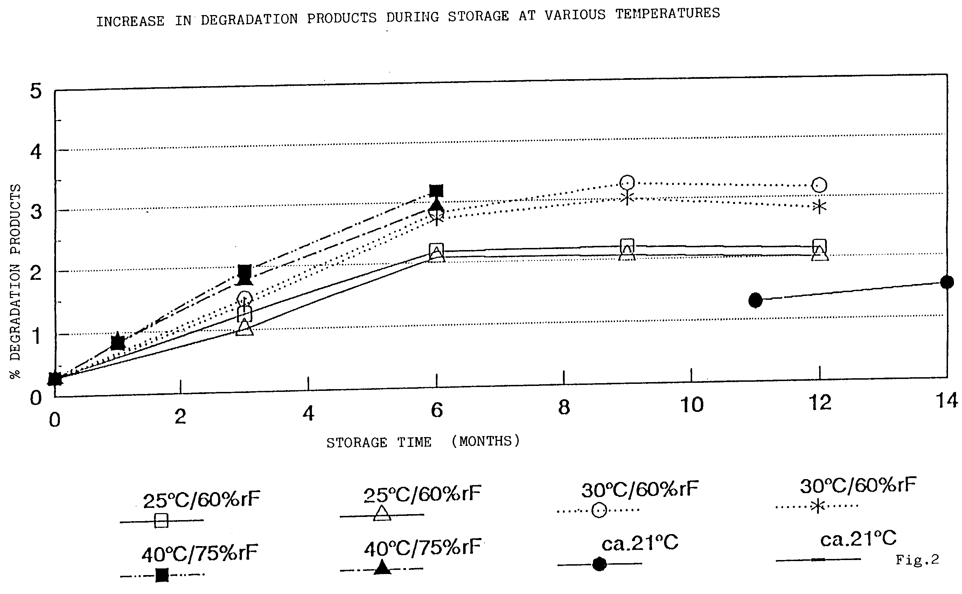

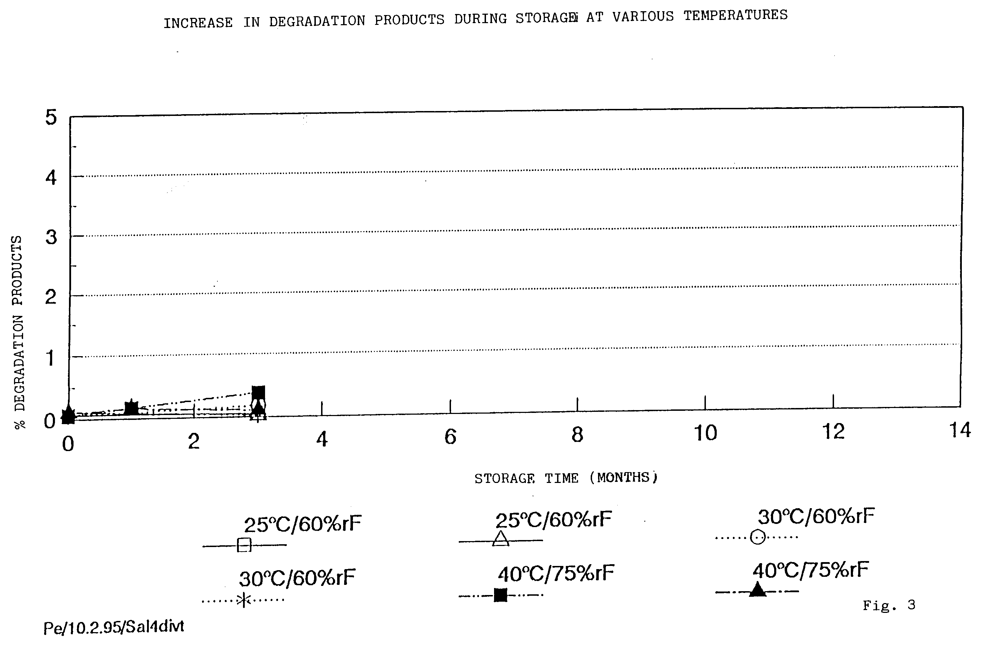

The results are shown in Figures 2 and 3. These results demonstrate the rate of formation of degradation products as well as the actual amounts formed are significantly higher in the tablets of the Comparative Example which contain cellulose derivatives.

A comparison of the amount of active ingredient which had degraded at one month and three months is shown also in the following table.

### Percentage of active ingredient degraded in tablet

| | According to the invention | Comparative Example |
|---|---|---|
| 25°C/60% rh | | |
| 1 month | 0.03% | 0.50% |
| 3 months | 0.03% | 1.00% |

| 40°C/75% rh | | |
|---|---|---|
| 1 month | 0.03% | 0.90% |
| 3 months | 0.40% | 1.80% |

## Claims

1. A solid, oral controlled release dosage form containing as active ingredient a pharmaceutically active compound which contains a 2-amino-1-hydroxyethyl-substituted benzene structure, wherein the amount of the initial pharmaceutically active compound which degrades after storage of the dosage form for three months at 40°C and 75% relative humidity is less than 1.5%.

2. A solid, oral controlled release dosage form according to claim 1 wherein the pharmaceutically active compound is dispersed in a controlled release matrix.

3. A solid, oral controlled release dosage form according to claim 2, wherein the controlled release matrix does not contain a cellulose derivative or a hydrogenated oil.

4. A solid, oral controlled release dosage form according to claim 3, wherein the controlled release matrix contains an aliphatic alcohol containing 8 to 18 carbon atoms optionally substituted by an aliphatic group containing 8 to 18 carbon atoms.

5. A solid, oral controlled release dosage form according to claim 4, wherein the aliphatic alcohol is one or more of lauryl alcohol, myristyl alcohol, cetyl alcohol, stearyl alcohol and cetostearyl alcohol.

6. A solid, oral controlled release dosage form according to any one of claims 2 to 6, wherein the controlled release matrix contains a dissacharide such as lactose.

7. A solid, oral controlled release dosage form according to any one of the preceding claims wherein the pharmaceutically active ingredient is a β₂-mimetic.

8. A solid, oral controlled release dosage form according to claim 7, wherein the β₂-mimetic is salbutamol or pharmaceutically acceptable salts thereof.

9. A solid, oral controlled release dosage form according to claim 8, wherein the salbutamol or salt thereof is dispersed in a matrix consisting of cetostearyl alcohol and lactose and optionally one or more conventional tabletting or capsulating excipients.

10. A composition according to any use of the preceding claims wherein the dissolution rate in vitro, when measured by 37°C is between 20% and 40% (by weight) of active ingredient released after one hour, between 30% and 60% (by weight) of active ingredient released after two hours, between 50% and 80% (by weight) of active ingredient released after four hours, between 75% and 95% (by weight) of active ingredient released after eight hours and more than 85% (by weight) released after ten hours.

11. A solid, oral controlled release dosage form according to any one of claims 7 to 10, in the form of a tablet suitable for twice a day dosing.
